# EUROPÄISCHE PATENTSCHRIFT

(11) **EP 2 990 398 B1**
(45) Veröffentlichungstag und Bekanntmachung des Hinweises auf die Patenterteilung: **19.12.2018**
(21) Anmeldenummer: 14182763.4
(22) Anmeldetag: 29.08.2014
(51) Int. Cl.: C07C 229/24, C09D 175/02, C08G 18/38

(54) **Hydrophile Polyasparaginsäureester**
Hydrophilic polyaspartic acid ester
Ester d'acide polyasparaginique hydrophile

(43) Veröffentlichungstag der Anmeldung: 02.03.2016
(73) Patentinhaber: Covestro Deutschland AG, 51373 Leverkusen (DE)
(72) Erfinder: Laas, Hans-Josef, 51519 Odenthal (DE); Grahl, Michael, 51371 Leverkusen (DE); Fleck, Olaf, 51467 Bergisch Gladbach (DE); Meier-Westhues, Hans-Ulrich, 51379 Leverkusen (DE); La Faille, Michèl, 4707 TH Roosendaal (NL)
(74) Vertreter: Levpat

(56) Entgegenhaltungen:
- EP-A1- 0 743 332
- EP-A1- 1 616 889

## Beschreibung

Die Erfindung betrifft hydrophile Polyasparaginsäureester, Gemische, Lösungen oder Dispersionen dieser sowie aus den hydrophilen Polyasparaginsäureestern erhältliche Polyurethanharnstoffe. Ein weiterer Gegenstand der Erfindung ist ein Verfahren zur Herstellung eines Beschichtungsmittels aus den hydrophilen Polyasparaginsäureestern. Darüber hinaus sind ein, die hydrophilen Polyasparaginsäureester enthaltendes, 2-Komponenten-System, dessen Verwendung zur Herstellung einer Beschichtung auf einem Substrat und die damit beschichteten Substrate weitere Gegenstände der Erfindung.

Hydrophile Polyasparaginsäureester der eingangs beschriebenen Art werden in der EP-A 1 616 889 offenbart. Diese Veröffentlichung beschreibt Umsetzungsprodukte von Diaminen mit speziellen, Ethergruppen enthaltenden Maleinsäureestern als Bindemittelkomponente für wässrige Lacksysteme. Aufgrund der für die Herstellung benötigten drastischen Reaktionsbedingungen weisen die entsprechenden Maleinsäureester ebenso wie die aus ihnen erhältlichen hydrophilen Polyasparaginsäureester eine starke Eigenfarbe auf, was sie für die Mehrzahl von Lackanwendungen ungeeignet macht.

Daher war es die Aufgabe der vorliegenden Erfindung hydrophile Polyasparaginsäureester mit spektrofotometrisch nach DIN EN 1557 gemessenen Hazen-Farbzahlen von 5 bis 100 Hazen zur Verfügung zu stellen, die sich hervorragend in Wasser lösen oder dispergieren lassen und aus denen sich, insbesondere durch Umsetzung mit hydrophilen Polyisocyanaten, farblose Beschichtungen herstellen lassen.

Diese Aufgabe wird durch die erfindungsgemäßen hydrophilen Polyasparaginsäureester gelöst, die durch Umsetzung von wenigstens einer Polyaminkomponente A), umfassend eine Aminverbindung der Formel (I), 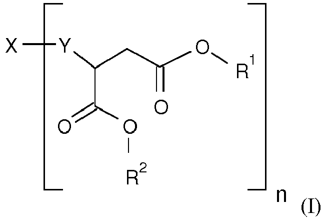 in welcher
- X: ein gesättigter oder ungesättigter, linearer oder verzweigter, aliphatischer oder cycloaliphatischer oder aromatischer organischer Rest ist, der substituiert oder unsubstituiert ist und/oder Heteroatome in der Kette aufweist,
- Y: eine an zwei Kohlenstoffatome gebundene sekundäre Aminogruppe ist,
- R¹ und R²: unabhängig voneinander gesättigte oder ungesättigte, lineare oder verzweigte, aliphatische oder cycloaliphatische oder aromatische organische Reste mit 1 bis 18 Kohlenstoffatomen sind, die substituiert oder unsubstituiert sind und/oder Heteroatome in der Kette aufweisen und
- n: eine natürliche Zahl von 1 bis 4 ist,
mit wenigstens einer Polyisocyanatkomponente B), umfassend ein Polyisocyanat, das wenigstens einen chemisch gebundenen Polyalkylenoxidpolyetheralkohol enthält, wobei die Anzahl der sekundären Aminogruppen Y zu der Anzahl der Isocyanatgruppen des Polyisocyanates in einem Verhältnis von 250:1 bis 3:1 steht, erhältlich sind.

Gemäß einer ersten bevorzugten Ausführungsform der Erfindung steht die Anzahl der sekundären Aminogruppen Y in der Formel (I) zu der Anzahl der Isocyanatgruppen des Polyisocyanates in einem Verhältnis von 200:1 bis 5:1, bevorzugt 150:1 bis 6:1 und besonders bevorzugt 120:1 bis 8:1.

Um eine verbesserte Löslichkeit in polaren Lösungsmitteln zu erreichen, weist das Polyisocyanat wenigstens einen chemisch gebundenen Polyalkylenoxidpolyetheralkohol auf.

Diese Polyalkylenoxidpolyetheralkohole können mono-, di- oder polyfunktionelle, bevorzugt mono- oder difunktionelle und besonders bevorzugt monofunktionelle Polyalkylenoxidpolyetheralkohole, wie sie beispielsweise durch Umsetzung geeigneter Startermoleküle mit wenigstens einem Alkylenoxid zugänglich sind, enthalten. Als Alkylenoxide sind beispielsweise Ethylenoxid und Propylenoxid geeignet. Die Startermoleküle können beliebige mono-, di- oder polyfunktionelle Alkohole des Molekulargewichtsbereiches 32 bis 300 sein, beispielsweise Methanol, Ethanol, n-Propanol, Isopropanol, n-Butanol, Isobutanol, sec-Butanol, die isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole, Hydroxymethylcyclohexan, 3-Methyl-3-hydroxymethyloxetan, 1,2-Ethandiol, 1,2- und 1,3-Propandiol, die isomeren Butandiole, Pentandiole, Hexandiole, Heptandiole und Octandiole, 1,2- und 1,4-Cyclohexandiol, 1,4-Cyclohexandimethanol, 4,4'-(1-Methylethyliden)-biscyclohexanol, 1,2,3-Propantriol, 1,1,1-Trimethylolethan, 1,2,6-Hexantriol, 1,1,1-Trimethylolpropan, 2,2-Bis(hydroxymethyl)-1,3-propandiol, 1,3,5-Tris(2-hydroxyethyl)-isocyanurat oder beliebige Gemische dieser Alkohole. Ganz besonders bevorzugte Polyalkylenoxidpolyetheralkohole sind solche, die unter Verwendung der obengenannten Monoalkohole des Molekulargewichtsbereiches 32 bis 150 als Startermoleküle hergestellt wurden.

Gemäß einer weiteren bevorzugten Ausführungsform umfasst der Polyalkylenoxidpolyetheralkohol wenigstens eine Polyalkylenoxidpolyethereinheit, die zu ≥ 40 mol-%, bevorzugt zu ≥ 50 mol-% und besonders bevorzugt zu ≥ 70 mol-% aus Ethylenoxideinheiten besteht und/oder im statistischen Mittel 5 bis 80, bevorzugt 5 bis 50 und besonders bevorzugt 5 bis 30 Ethylenoxideinheiten aufweist, umfasst. Hierdurch lässt sich eine sehr gute Löslichkeit oder Dispergierbarkeit der hydrophilen Polyasparaginsäureester in polaren Lösungsmitteln, bevorzugt in Wasser, erreichen. Für diese bevorzugte Ausführungsform bieten die erfindungsgemäßen hydrophilen Polyasparaginsäureester durch die hervorragende Dispergierbarkeit trotz vergleichsweise geringer Ethylenoxidgehalte einen besonderen Vorteil, da sich durch Verwendung dieser auf diese Weise Beschichtungen erhalten lassen, die aufgrund des niedrigen Gehaltes an hydrophilen Gruppen insbesondere eine ausgezeichnete Wasserfestigkeit aufweisen.

In einer besonders bevorzugten Ausführungsform enthält die Polyalkylenoxidpolyethereinheit reine Polyethylenglycolmonomethyletheralkohole, die im statistischen Mittel 5 bis 80, bevorzugt 5 bis 50 und besonders bevorzugt 5 bis 30 Ethylenoxideinheiten aufweisen. Dadurch wird die Löslichkeit oder Dispergierbarkeit der erfindungsgemäßen hydrophilen Polyasparaginsäureester noch weiter verbessert.

In Weiterbildung der Erfindung ist vorgesehen, dass das Polyisocyanat wenigstens ein aliphatisches, cycloaliphatisches, araliphatisches oder aromatisches, bevorzugt wenigstens ein aliphatisches oder cycloaliphatisches Polyisocyanat sein kann.

Hierbei ist es weiterhin bevorzugt, dass das Polyisocyanat neben wenigstens einer chemisch gebundenen, nicht-ionischen, hydrophilen Gruppe als eine weitere Aufbaukomponente ein oder mehrere Diisocyanate des Molekulargewichtsbereiches 140 bis 400 enthalten kann. Bevorzugte Diisocyanate sind 1,4-Diisocyanatobutan, 1,5-Diisocyanatopentan, 1,6-Diisocyanatohexan (HDI), 1,5-Diisocyanato-2,2-dimethylpentan, 2,2,4- bzw. 2,4,4-Trimethyl-1,6-diisocyanatohexan, 1,10-Diisocyanatodecan, 1,3- und 1,4-Diisocyanatocyclohexan, 1,4-Diisocyanato-3,3,5-trimethylcyclohexan, 1,3-Diisocyanato-2-methylcyclohexan, 1,3-Diisocyanato-4-methylcyclohexan, 1-Isocyanato-3,3,5-trimethyl-5-isocyanatomethylcyclohexan (Isophorondiisocyanat; IPDI), 1-Isocyanato-1-methyl-4(3)-isocyanatomethylcyclohexan, 2,4'- und 4,4'-Diisocyanatodicyclohexylmethan (H12-MDI), 1,3- und 1,4-Bis(isocyanatomethyl)cyclohexan, 4,4'-Diisocyanato-3,3'-dimethyldicyclohexylmethan, 4,4'-Diisocyanato-3,3',5,5'-tetramethyldicyclohexylmethan, 4,4'-Diisocyanato-1,1'-bi(cyclohexyl), 4,4'-Diisocyanato-3,3'-dimethyl-1,1'-bi(cyclohexyl), 4,4'-Diisocyanato-2,2',5,5'-tetra-methyl-1,1'-bi(cyclohexyl), 1,8-Diisocyanato-p-menthan, 1,3-Diisocyanatoadamantan, 1,3-Dimethyl-5,7-diisocyanatoadamantan, 1,3- und 1,4-Bis-(isocyanatomethyl)benzol (XDI), 1,3- und 1,4-Bis(1-isocyanato-1-methylethyl)-benzol (TMXDI), Bis(4-(1-isocyanato-1-methylethyl)phenyl)-carbonat, 1,3- und 1,4-Phenylendiisocyanat, 2,4- und 2,6-Toluylendiisocyanat (TDI) sowie beliebige Gemische dieser Isomeren, Diphenylmethan-2,4'- und/oder -4,4'-diisocyanat (MDI) und Naphthylen-1,5-diisocyanat (NDI). Weitere ebenfalls geeignete Diisocyanate finden sich darüber hinaus beispielsweise in Justus Liebigs Annalen der Chemie Band 562 (1949) S. 75 - 136.

Besonders bevorzugte Diisocyanate sind 1,5-Diisocyanatopentan, 1,6-Diisocyanatohexan (HDI), 1-Isocyanato-3,3,5-trimethyl-5-isocyanatomethylcyclohexan (Isophorondiisocyanat; IPDI) und/oder 2,4'- und 4,4'-Diisocyanatodicyclohexylmethan (H12-MDI).

In einer weiteren bevorzugten Ausführungsform können beim Einsatz aliphatischer und/oder cycloaliphatischer Diisocyanate auch Diisocyanate mit aromatisch gebundenen Isocyanatgruppen in unterschüssig molarer Menge bezogen auf die Gesamtmenge an freien Isocyanatgruppen, ebenfalls enthalten sein. Derartige gemischt aliphatisch/aromatische Polyisocyanate sind bereits bekannt und beispielsweise gemäß EP-A 0 680 983 durch Umsetzung von Polyethylenoxidpolyethern mit Mischungen monomerenarmer Polyisocyanate auf Basis von HDI und solchen auf Basis von 2,4(6)-Diisocyanatotoluol (Toluylendiisocyanat, TDI) zugänglich.

Gemäß einer weiteren bevorzugten Ausführungsform enthält das Polyisocyanat Allophanatgruppen und weist gegebenenfalls bis zu 10 mol-%, bevorzugt bis zu 5 mol-%, an Isocyanurat- und/oder Uretdiongruppen, bezogen auf die Gesamtmenge an Allophanat-, Isocyanurat- und Uretdionstrukturen, auf.

In einer weiteren besonders bevorzugten Ausführungsform weist das Polyisocyanat eine mittlere Isocyanatfunktionalität von 1,0 bis 3,0, bevorzugt von 1,5 bis 2,5 und besonders bevorzugt von 1,8 bis 2,2, einen Gehalt an Isocyanatgruppen von 3 bis 25 Gew.-%, bevorzugt 4 bis 20 Gew.-% und besonders bevorzugt von 5 bis 15 Gew.-%, sowie an innerhalb von Polyalkylenoxidpolyethereinheiten gebundenen Ethylenoxideinheiten von 5 bis 80 Gew.-%, bevorzugt von 5 bis 75 Gew.-% und besonders bevorzugt von 10 bis 70 Gew.-%, auf, wobei die Polyalkylenoxidpolyethereinheiten im statistischen Mittel 5 bis 80, bevorzugt 5 bis 50 und besonders bevorzugt 5 bis 30 Ethylenoxideinheiten aufweist.

Hinsichtlich der Aminverbindung der Formel (I) ist in einer weiteren bevorzugten Ausführungsform vorgesehen, dass n in der Formel (I) eine natürliche Zahl von 1 bis 3, bevorzugt 1 oder 2 und besonders bevorzugt 2 ist.

Es ist ebenfalls bevorzugt, wenn X in der Formel (I) ein organischer Rest mit einem Molekulargewicht von 28 g/mol bis 6000 g/mol, bevorzugt 45 bis 5000 g/mol und besonders bevorzugt 84 bis 500 g/mol ist.

Gemäß einer weiteren bevorzugten Ausführungsform ist X in der Formel (I) aus einer Gruppe von organischen Resten ausgewählt, die erhalten wird, wenn die Aminogruppen der Verbindungen mit einem Molekulargewicht von 60 bis 238 g/mol, wie z. B. Ethylendiamin, 1,2-Diaminopropan, 1,4-Diaminobutan, 1,5-Diamino-2-methylpentan (Dytek® A der Fa. DuPont), 1,6-Diaminohexan, 2,5-Diamino-2,5-dimethylhexan, 2,2,4- und/oder 2,4,4-Trimethyl-1,6-diaminohexan, 1,11-Diaminoundecan, 1,12-Diaminododecan, 1-Amino-3,3,5-trimethyl-5-aminomethylcyclohexan (IPDA), 1,3-Diamino-2- und/oder -4-methylcyclohexan, Isopropyl-2,4- und/oder 2,6-diaminocyclohexan, 1,3-Bis-(aminomethyl)-cyclohexan, 2,4'- und/oder 4,4'-Diamino-dicyclohexylmethan, 3,3'-Dimethyl-4,4'-diamino-dicyclohexylmethan (Laromin® C 260; BASF SE, Ludwigshafen, DE), die isomeren, eine Methylgruppe als Kernsubstituenten aufweisenden Diaminodicyclohexylmethane (C-Monomethyl-diaminodicyclohexylmethane), 3(4)-Aminomethyl-1-methylcyclohexylamin und 1,3-Bis-(aminomethyl)-benzol, entfernt werden und n für die natürliche Zahl 2 steht, geeignet.

In einer weiteren bevorzugten Ausführungsform ist X in der Formel (I) aus einer Gruppe von organischen Resten ausgewählt, die erhalten wird, wenn die Aminogruppen von Verbindungen, die wenigstens eine von Aminogruppen verschiedene und im Temperaturbereich von 20 bis 200°C gegenüber Isocyanatgruppen reaktive Gruppen enthalten, entfernt werden und n für die natürliche Zahl 1 oder 2 steht. Hierbei kann es sich beispielsweise um Aminoalkohole, wie z. B. 2-Aminoethanol, die isomeren Aminopropanole und -butanole, 3-Amino-1,2-propandiol und 1,3-Diamino-2-propanol handeln.

Für den Fall, das n in der Formel (I) für die natürliche Zahl 3 oder 4 steht, kann X beispielsweise aus einer Gruppe von organischen Resten ausgewählt werden, die erhalten wird, wenn die Aminogruppen der Verbindungen 4-Aminomethyl-1,8-octandiamin, 2,2',2"-Triaminotriethylamin, 1,3,5-Tris-(aminomethyl)-2,4,6-triethylbenzol, Tris-1,1,1-aminoethylethan, 1,2,3-Triaminopropan, Tris-(3-aminopropyl)-amin und N,N,N',N'-Tetrakis-(2-aminoethyl)-ethylendiamin entfernt werden. Auch niedermolekulare Polyetherpolyamine mit aliphatisch gebundenen primären Aminogruppen, wie sie beispielsweise unter der Bezeichnung Jeffamin® (Huntsman Performance Products, The Woodlands, Texas, USA) vertrieben werden, sind hierbei prinzipiell geeignete Verbindungen.

Besonders bevorzugt ist X in der Formel (I) aus einer Gruppe von organischen Resten ausgewählt, die erhalten wird, wenn die Aminogruppen der Verbindungen 1,5-Diamino-2-methylpentan (Dytek® A der Fa. DuPont), 2,4'-Diamino-dicyclohexylmethan, 4,4'-Diamino-dicyclohexylmethan, 3,3'-Dimethyl-4,4'-diamino-dicyclohexylmethan (Laromin® C 260; BASF SE, Ludwigshafen, DE) oder Polypropylenglykol-diaminen des Molekulargewichtsbereiches 200 bis 500 g/mol entfernt werden und n die natürliche Zahl 2 ist. Geeignete Propylenglykol-diamine sind in diesem Zusammenhang beispielsweise Mischungen aus Jeffamin® D-230 und Jeffamin® D-400 (Huntsman Performance Products, The Woodlands, Texas, USA).

In einer weiteren bevorzugten Ausführungsform sind R¹ und R² in der Formel (I) unabhängig voneinander aliphatische organische Reste mit 1 bis 9 Kohlenstoffatomen, bevorzugt unabhängig voneinander Methyl-, Ethyl-, n-Butyl- und/oder 2-Ethylhexylreste und besonders bevorzugt Ethylreste.

Gemäß einer weiteren bevorzugten Ausführungsform enthält das erfindungsgemäße hydrophile Polyasparaginsäureestergemisch Verbindungen, die sekundäre Aminogruppen und/oder Harnstoffgruppen aufweisen, wobei das Gemisch ein Verhältnis der Anzahl der sekundären Aminogruppen zu der Anzahl der Harnstoffgruppen 249:1 bis 2:1, bevorzugt 199:1 bis 4:1, besonders bevorzugt 149:1 bis 5:1 und ganz besonders bevorzugt 119:1 bis 7:1 aufweist.

Die erfindungsgemäßen hydrophilen Polyasparaginsäureester können auch durch den Begriff farbhell beschrieben werden, wobei der Begriff farbhell im Rahmen der vorliegenden Erfindung durch oben angegebenen Bereich der Hazen-Farbzahl definiert ist.

Die generelle Herstellung von Aminverbindungen der Formel (I) kann beispielsweise, wie in EP-A 0 403 921, EP-A 0 689 881 oder EP-A 0 816 326 beschrieben, durch Reaktion einer primäre Aminogruppen enthaltenden Verbindung der Formel (II) 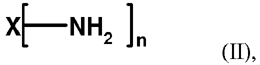 in welcher X und n der in der Formel (I) angegebenen Definition entsprechen, mit Fumarsäureestern und/oder Maleinsäureestern der Formel (III)

**R¹OOC-CH=CH-COOR²** (III)

in welcher R¹ und R² der in der Formel (I) angegebenen Definition entsprechen, erfolgen.

Generell können die genannten Verbindungen der Formel (II) sowohl einzeln als auch in Form beliebiger Gemische untereinander zur Herstellung von Aminverbindungen der Formel (I) eingesetzt werden. Auch die Fumarsäureester und/oder Maleinsäureester der Formel (III) können sowohl einzeln als auch in Form beliebiger Gemische untereinander zur Herstellung von Aminverbindungen der Formel (I) eingesetzt werden.

Die Herstellung der Aminverbindung der Formel (I) kann in Gegenwart eines organischen Lösungsmittels erfolgen. Geeignete Lösungsmittel hierfür sind insbesondere solche, die sich gegenüber den reaktiven Gruppen der Ausgangskomponenten inert verhalten, beispielsweise die üblichen Lacklösungsmittel, wie z. B. Ethylacetat, Butylacetat, Ethylenglykolmonomethyl- oder - ethyletheracetat, 1-Methoxypropyl-2-acetat, 3-Methoxy-n-butylacetat, Aceton, 2-Butanon, 4-Methyl-2-pentanon, Cyclohexanon, Toluol, Xylol, Chlorbenzol, Testbenzin, höher substituierte Aromaten, wie sie beispielsweise unter den Bezeichnungen Solventnaphtha, Solvesso®, Isopar®, Nappar® (Deutsche EXXON CHEMICAL GmbH, Köln, DE) und Shellsol® (Deutsche Shell Chemie GmbH, Eschborn, DE) im Handel sind, aber auch Lösungsmittel wie Propylenglykoldiacetat, Diethylenglykoldimethylether, Dipropylenglykoldimethylether, Diethylenglykolethyl- und -butyletheracetat, N-Methylpyrrolidon und N-Methylcaprolactam, oder beliebige Gemische solcher Lösungsmittel. Besonders bevorzugt ist jedoch, dass die Herstellung der Aminverbindung der Formel (I) ohne Mitverwendung organischer Lösungsmittel durchgeführt wird. Dadurch können eventuelle Rückstände organischer Lösungsmittel in den Aminverbindungen reduziert oder sogar ganz vermieden werden.

Unabhängig von der eventuellen Mitverwendung eines Lösungsmittels kann die Herstellung der Aminverbindung der Formel (I) bevorzugt durch äquimolare Umsetzung wenigstens einer Verbindung der Formel (II) mit wenigstens einem Fumarsäureester oder Maleinsäureester der Formel (III) erfolgen. Zur gezielten Variation anwendungstechnischer Eigenschaften kann das Äquivalenzverhältnis von Maleinsäureester und/oder Fumarsäureester der Formel (III) zu Aminogruppen der Verbindung der Formel (II) von 1,2:1 bis 1:2 variiert werden.

Die Herstellung der oben angegebenen Diisocyanate kann nach bekannten Verfahren, z. B. durch Phosgenierung oder auf phosgenfreiem Weg, beispielsweise durch Urethanspaltung, erfolgen. Aus den angegebenen Diisocyanaten lassen sich beispielsweise Polyisocyanate mit Uretdion-, Isocyanurat-, Allophanat-, Biuret-, Iminooxadiazindion- und/oder Oxadiazintrionstruktur herstellen, wie sie unter anderem in J. Prakt. Chem. 336 (1994) 185 - 200, in DE-A 1 670 666, DE-A 1 954 093, DE-A 2 414 413, DE-A 2 452 532, DE-A 2 641 380, DE-A 3 700 209, DE-A 3 900 053 und DE-A 3 928 503 oder in EP-A 0 336 205, EP-A 0 339 396 und EP-A 0 798 299 beschrieben sind.

Zur Einführung der Polyalkylenoxidpolyethereinheiten in das Polyisocyanat können die vorstehend genannten Polyisocyanate beispielsweise mit gegenüber Isocyanatgruppen reaktiven Gruppen aufweisenden Polyalkylenoxidpolyetheralkoholen umgesetzt werden. Die Herstellung der solcher Polyisocyanate kann beispielsweise nach den in der EP-A 206 059, EP-A 540 985 oder US-P 5 200 489 beschriebenen Verfahren durch Umsetzung monomerenarmer Polyisocyanate mit aliphatisch und/oder cycloaliphatisch gebundenen Isocyanatgruppen mit hydrophilen monofunktionellen Polyalkylenoxidpolyetheralkoholen der oben genannten Art erfolgen.

Die Allophanatgruppen enthaltenden Polyisocyanate können beispielsweise nach den in der EP-A 0 524 500, EP-A 0 566 037 oder US-P 5 086 175 beschriebenen Verfahren durch Umsetzung einfacher aliphatischer und/oder cycloaliphatischer Diisocyanate mit Polyalkylenoxidpolyetheralkoholen der oben genannten Art unter Einhaltung eines NCO/OH-Äquivalentverhältnisses von 5:1 bis 50:1 in Gegenwart einer die Bildung von Allophanatgruppen und gleichzeitig die Trimerisierung und/oder Dimerisierung von Isocyanatgruppen beschleunigenden Verbindung erhalten werden. Nach der Reaktion kann das nicht umgesetzte monomere Diisocyanat bis auf einen Restgehalt von weniger als 0,5 Gew.-% abgetrennt werden.

In diesem Zusammenhang besonders bevorzugte Polyisocyanate sind difunktionelle Allophanatgruppen enthaltende und gegebenenfalls Isocyanurat- und/oder Uretdiongruppen aufweisende Polyisocyanate, auf Basis aliphatischer und/oder cycloaliphatischer Diisocyanate, die beispielsweise nach dem Verfahren der EP-A 0 682 012 oder wie in der EP-A 0 850 896 beispielhaft beschrieben, durch Umsetzung monofunktioneller Polyetheralkohole der oben genannten Art mit einem molaren Diisocyanatüberschuß in Gegenwart von Allophanatisierungkatalysatoren und anschließende Abtrennung des nicht umgesetzten monomeren Diisocyanates erhältlich sind.

Gegebenenfalls Isocyanurat- und/oder Uretdiongruppen aufweisend bedeutet in diesem Zusammenhang, dass die als Polyisocyanatkomponente B) besonders geeigneten Allophanatpolyisocyanate maximal bis zu 10 mol-%, vorzugsweise bis zu 5 mol-%, an Isocyanurat- und/oder Uretdiongruppen, bezogen auf die Gesamtmenge an Allophanat-, Isocyanurat- und Uretdiongruppen, enthalten kann.

Das Verhältnis an Allophanat-, Isocyanurat- und/oder Uretdiongruppen kann z. B. durch eine NMR-spektroskopische Untersuchung bestimmt werden. Bevorzugt lässt sich hierbei die ¹³C-NMR-Spektroskopie, vorzugsweise protonenentkoppelt, einsetzen, da die Allophanat-, Isocyanurat- und/oder Uretdiongruppen charakteristische Signale liefern.

Zur Herstellung der erfindungsgemäßen hydrophilen Polyasparaginsäureester kann die Polyaminkomponente A) mit der Polyisocyanatkomponente B) gemäß einer weiteren bevorzugten Ausführungsform bei Temperaturen von 0 bis 120°C, bevorzugt von 20 bis 80°C, besonders bevorzugt von 20 bis 60°C umgesetzt werden.

Die Herstellung der erfindungsgemäßen hydrophilen Polyasparaginsäureester kann in Gegenwart wenigstens eines gegenüber den reaktiven Gruppen der Aminverbindung der Formel (I) und der Polyisocyanate inerten Lösungsmittels erfolgen. Hierbei handelt es sich beispielsweise um die üblichen Lacklösungsmittel, wie sie bereits als gegebenenfalls bei der Herstellung der Aminverbindung der Formel (I) mitzuverwendende Lösungsmittel beschrieben sind. Derartige Lösungsmittel kommen, falls gewünscht, in einer Menge von beispielsweise bis zu 70 Gew.-%, bevorzugt bis zu 60 Gew.-%, besonders bevorzugt bis zu 50 Gew.-% zum Einsatz. Bevorzugt ist jedoch, dass die Herstellung der erfindungsgemäßen hydrophilen Polyasparaginsäureester ohne Zusatz von Lösungsmittel erfolgt. Im letzteren Fall zeichnen sich die erfindungsgemäßen hydrophilen Polyasparaginsäureester durch einen niedrigen Gehalt an flüchtigen organischen Verbindungen (VOC-Gehalt) aus.

In einer weiteren bevorzugten Ausführungsform kann beim Verfahren zur Herstellung der erfindungsgemäßen hydrophilen Polyasparaginsäureester die Polyaminkomponente A), gegebenenfalls unter Inertgas, wie beispielsweise Stickstoff, und gegebenenfalls in Gegenwart eines Lösungsmittels der oben genannten Art, bei einer Temperatur zwischen 0 und 100°C vorgelegt werden. Anschließend kann die Polyisocyanatkomponente B), gegebenenfalls gemeinsam mit weiterem Lösungsmittel so zugegeben werden, dass die Temperatur des Reaktionsgemisches 120°C nicht übersteigt.

Unabhängig von der jeweiligen Ausführungsform kann der Verlauf der Umsetzung der Polyaminkomponente A) mit der Polyisocyanatkomponente B) beispielsweise IR-spektroskopisch verfolgt werden. Nach vollständiger Reaktion von Isocyanat- und Aminogruppen, erkennbar beispielsweise am Verschwinden der Isocyanatbande bei ca. 2270 cm⁻¹ im IR-Spektrum, erhält man als Produkte die erfindungsgemäßen hydrophilen Polyasparaginsäureester in Form klarer farbheller Harze, die in Abhängigkeit von der Art und Menge der gewählten Ausgangsverbindungen in lösemittelfreier Form bei 23°C üblicherweise Viskositäten von 1000 bis 20000 mPas aufweisen.

Die erfindungsgemäßen hydrophilen Polyasparaginsäureester sind für eine Vielzahl von Anwendungen geeignet. So lassen sie sich beispielsweise in einem Lösungsmittel lösen oder dispergieren. Als Lösungsmittel sind beispielsweise die bei der Herstellung der Aminverbindung der Formel (I) beschriebenen bekannten üblichen Lacklösungsmittel geeignet. Daher sind ein weiterer Gegenstand der Erfindung Lösungen oder Dispersionen enthaltend wenigstens einen erfindungsgemäßen hydrophilen Polyasparaginsäureester.

Gemäß einer weiteren bevorzugten Ausführungsform der erfindungsgemäßen Lösungen oder Dispersionen der hydrophilen Polyasparaginsäureester ist Wasser als Lösungsmittel enthalten und bevorzugt ist Wasser das alleinige Lösungsmittel. Somit ist nach dieser bevorzugten Ausführungsform wenigstens ein weiterer Bestandteil dieser Lösungen oder Dispersionen Wasser. Besonders bevorzugt ist hierbei weiterhin, wenn Wasser als alleiniges Lösungsmittel eingesetzt wird. Die wässrigen Lösungen oder Dispersionen lassen sich leicht, ohne Einsatz hoher Scherkräfte durch bloßes Einrühren der erfindungsgemäßen hydrophilen Polyasparaginsäureester in Wasser herstellen. Ein Vorteil ist hierbei, dass durch den Einsatz von Wasser als Lösungsmittel die Verwendung umweltschädigender Substanzen reduziert werden kann.

Gemäß einer weiteren bevorzugten Ausführungsform weisen die vorzugsweise wässrigen Lösungen oder Dispersionen Festkörpergehalte von 10 bis 60 Gew.-%, bevorzugt 20 bis 50 Gew.-%, besonders bevorzugt 30 bis 45 Gew.-% auf. Ein Vorteil ist, dass solche Lösungen oder Dispersionen eine sehr gute Sedimentationstabilität aufweisen.

Da die erfindungsgemäßen hydrophilen Polyasparaginsäureester sowohl lösungsmittelfrei als auch in Form obiger Lösungen oder Dispersionen über freie sekundäre Aminogruppen verfügen, sind sie beispielsweise für die Umsetzung mit NCO-funktionellen Vernetzungsmitteln sehr gut geeignet.

Daher ist ein weiterer Gegenstand der Erfindung ein Polyurethanharnstoff, erhältlich durch Umsetzung wenigstens eines erfindungsgemäßen hydrophilen Polyasparaginsäureesters mit wenigstens einem NCO-funktionellen Vernetzungsmittel. Dabei kann die Herstellung von Polyharnstoffen nach dem Isocyanat-Polyadditionsverfahren erfolgen.

Als NCO-funktionelle Vernetzungsmittel können beliebige hydrophobe und/oder hydrophil modifizierte Polyisocyanate, die unblockiert oder in mit den aus der Polyurethanchemie bekannten Blockierungsmitteln blockiert eingesetzt werden können, zum Einsatz kommen. Besonders bevorzugt kommen hierbei hydrophobe und/oder hydrophil modifizierte Polyisocyanate mit einer nach DIN EN ISO 3219 bestimmten Viskosität von ≤ 3000 mPas, bevorzugt ≤ 2000 mPas, bei 23°C zum Einsatz. Gegebenenfalls können diese auch als wässrige Lösung oder Dispersion eingesetzt werden.

Geeignete hydrophobe Polyisocyanate sind beispielsweise neben den vorstehend genannten Polyisocyanaten, die wenigstens eine chemisch gebundene, nicht-ionische, hydrophile Gruppe enthaltenauch, durch Modifizierung einfacher Diisocyanate erhältliche, hydrophobe Polyisocyanate mit Uretdion-, Isocyanurat-, Allophanat-, Biuret-, Iminooxadiazindion- und/oder Oxadiazintrionstruktur, wobei solche mit nach DIN EN ISO 3219 bestimmten Viskositäten bei 23°C von ≤ 3000 mPas, bevorzugt ≤ 2000 mPas, bevorzugt sind.

Geeignete hydrophil modifizierte Polyisocyanate sind beispielsweise die vorstehend genannten wenigstens eine chemisch gebundene, nicht-ionische, hydrophile Gruppe enthaltenden Polyisocyanate. Weitere geeignete Polyisocyanate können auch die nach den Verfahren der EP-A 0 959 087 und EP-A 1 276 787 durch Umsetzung monomerenarmer, aus mindestens zwei Diisocyanatmolekülen bestehenden Polyisocyanaten mit monofunktionellen Polyethylenoxidpolyetheralkoholen unter Allophanatisierung erhältlichen Polyisocyanatgemische sein. Ebenfalls geeignet sind die aus der WO 01/88006 bekannten, durch Umsetzung hydrophober Polyisocyanate mit 2-(Cyclohexylamino)-ethansulfonsäure und/oder 3-(Cyclohexylamino)-propansulfonsäure erhältlichen ionisch-modifizierten, sulfonatgruppenhaltigen Polyisocyanatgemische.

Die vorstehend beschriebenen hydrophoben und/oder hydrophil modifizierten Polyisocyanate können auch in mit aus der Polyurethanchemie bekannten Blockierungsmitteln blockierter Form im Sinne von 1-Komponenten-Systemen eingesetzt werden. Geeignete Blockierungsmittel sind beispielsweise Malonsäurediethylester, Acetessigester, aktivierte cyclische Ketone, wie z. B. Cyclopentanon-2-carboxymethylester und -carboxyethylester, Acetonoxim, Butanonoxim,-Caprolactam, 3,5-Dimethylpyrazol, 1,2,4-Triazol, Dimethyl-1,2,4-triazol, Imidazol, Benzyl-tert.-butylamin oder beliebige Gemische dieser Blockierungsmittel. Für den Fall das ein Lösungsmittel bei der Verwendung derartig blockierter Polyisocyanate eingesetzt werden soll, ist die Verwendung von Wasser besonders bevorzugt.

Schließlich können auch durch physikalische Abmischung externer Emulgatoren mit hydrophoben Polyisocyanaten der obengenannten Art hydrophil modifizierte Polyisocyanatgemische erhalten werden, die als NCO-funktionelle Vernetzungsmittel für die erfindungsgemäßen hydrophilen Polyasparaginsäureester geeignet sind.

Derartige externe Emulgatoren können beispielsweise isocyanatgruppenfreie Umsetzungsprodukte monomerer Diisocyanate oder Diisocyanatgemische mit den oben als zur Herstellung der Polyisocyanatkomponenten B) genannten geeigneten mono- oder polyfunktionellen Polyetheralkoholen, insbesondere mit reinen Polyethylenglycolmonomethyletheralkoholen, wie sie beispielsweise in EP-B 0 486 881 beschrieben sind, sein.

Ein weiterer Typ geeigneter externer Emulgatoren zur Modifizierung hydrophober Polyisocyanate sind solche, die in einem Molekül gleichzeitig ionische und nichtionische Strukturen enthalten. Bei diesen Emulgatoren kann es sich beispielsweise um mit tertiären Aminen neutralisierte Alkylphenolpolyglykolether-phosphate und -phosphonate oder Fettalkoholpolyglykoletherphosphate und -phosphonate, wie sie beispielsweise in WO 97/31960 zur Hydrophilierung von Polyisocyanaten beschrieben sind, oder auch um mit solchen tertiären Aminen neutralisierte Alkylphenolpolyglykolether-sulfate oder Fettalkoholpolyglykolether-sulfate handeln. Sowohl die erfindungsgemäßen hydrophilen Polyasparaginsäureester selbst als auch die Gemische und/oder Lösungen oder Dispersionen dieser sind beispielsweise hervorragend als Bindemittel zur Formulierung von Lacken und Beschichtungsmitteln, bevorzugt zur Formulierung von wässrigen Lacken und/oder wässrigen Beschichtungsmitteln geeignet.

Daher ist ein Verfahren zur Herstellung eines Beschichtungsmittels ein weiterer Gegenstand der Erfindung. Bei diesem Verfahren wird wenigstens ein erfindungsgemäßer hydrophiler Polyasparaginsäureester oder wenigstens eine Lösung oder Dispersion der hydrophilen Polyasparaginsäureester mit wenigstens einem NCO-funktionellen Vernetzungsmittel vermischt. Hierbei werden besonders bevorzugt wässrige Lösungen oder Dispersionen der erfindungsgemäßen Polyasparaginsäureester eingesetzt. In einer weiteren besonders bevorzugten Ausführungsform werden niedrigviskose hydrophobe, insbesondere solche mit einer bei 23°C nach DIN EN ISO 3219 bestimmten Viskosität von ≤ 2000 mPas, und/oder hydrophil modifizierte Polyisocyanate als NCO-funktionelle Vernetzungsmittel eingesetzt. Diese können neben einem Einsatz ohne Lösungsmittel auch als Lösungen oder Dispersionen verwendet werden.

Bei der Herstellung der Lacke und Beschichtungsmittel aus den erfindungsgemäßen hydrophilen Polyasparaginsäureestern bzw. deren Lösungen oder Dispersionen, bevorzugt wässrigen Dispersionen, werden die genannten NCO-funktionellen Vernetzungsmittel im allgemeinen in solchen Mengen eingesetzt, die einem Äquivalentverhältnis von Aminogruppen zu gegebenenfalls blockierten Isocyanatgruppen von 2:1 bis 0,5:1, bevorzugt von 1,5:1 bis 0,8:1, besonders bevorzugt von 1,1:1 bis 0,9:1 entsprechen.

Das Vermischen der erfindungsgemäßen hydrophilen Polyasparaginsäureester mit dem NCOfunktionellem Vernetzungsmittel, welches im Falle der Verwendung hydrophil modifizierter Polyisocyanate gegebenenfalls in Wasser voremulgiert vorliegen kann, kann durch einfaches Verrühren vor der Verarbeitung der Beschichtungsmittel nach beliebigen Methoden, durch Verwendung von dem Fachmann bekannten mechanischen Hilfsmitteln oder auch unter Verwendung von 2-Komponenten-Spritzpistolen erfolgen.

Die Lösungen oder Dispersionen der erfindungsgemäßen hydrophilen Polyasparaginsäureester, bevorzugt die wässrigen Lösungen oder Dispersionen, können sowohl als alleiniges Bindemittel als auch in Kombination mit weiteren gegebenenfalls in Wasser gelösten oder dispergierten Lackbindemittel oder Lackbindemittelkomponenten, die vorzugsweise ebenfalls gegenüber Isocyanatgruppen reaktionsfähige Gruppen tragen, eingesetzt werden.

Geeignete Kombinationspartner für die Lösungen oder Dispersionen der erfindungsgemäßen hydrophilen Polyasparaginsäureester, die bei der Formulierung der erfindungsgemäßen Beschichtungsmittel mitverwendet werden können, sind beispielsweise Polyacrylate, insbesondere solche des zahlenmittleren Molekulargewichtsbereichs 1000 bis 10000 g/mol, gegebenenfalls Urethangruppen enthaltende, hydroxyfunktionelle Polyesterharze der aus der Polyester- und Alkydharzchemie bekannten Art, hydroxyfunktionelle Polyurethane aber auch Polyurethane oder Polyharnstoffe, die aufgrund der in den Urethan- bzw. Harnstoffgruppen vorliegenden aktiven Wasserstoffatome mit Polyisocyanaten vernetzbar sind.

Hierbei ist es bevorzugt, wenn wässrige Lösungen oder wässrige Dispersionen der erfindungsgemäßen hydrophilen Polyasparaginsäureester verwendet werden und/oder die Kombinationspartner in wässrigem Lösungsmittel vorliegen.

Die Herstellung der erfindungsgemäßen Beschichtungsmittel kann unter Mitverwendung organischer Lösungsmittel erfolgen. Geeignete Lösungsmittel sind beispielsweise die üblichen Lacklösungsmittel, wie sie bereits bei der Herstellung der Aminverbindung der Formel (I) und/oder der Herstellung der erfindungsgemäßen hydrophilen Polyasparaginsäureester als gegebenenfalls mitzuverwendende Lösungsmittel beschrieben sind, oder beliebige Gemische solcher Lösungsmittel in untergeordeneten Mengen, beispielsweise in Mengen von bis zu 30 Gew.-%, vorzugsweise bis zu 20 Gew.-%, besonders bevorzugt bis zu 10 Gew.-%, bezogen auf die Menge an Polyasparaginsäureester. Die Lösungsmittel können den erfindungsgemäßen hydrophilen Polyasparaginsäureestern und/oder dem NCO-funktionellen Vernetzungsmittel vor der Vereinigung der beiden Komponenten zugesetzt werden. Gegebenenfalls können die erfindungsgemäßen wässrigen Lösungen oder Dispersionen der erfindungsgemäßen hydrophilen Polyasparaginsäureester in untergeordneten Mengen auch nichtfunktionelle wässrige Lackbindemittel zur Erzielung ganz spezieller Eigenschaften, beispielsweise als Additiv zur Haftverbesserung zugemischt werden. Bevorzugt ist jedoch, dass die Herstellung der erfindungsgemäßen Beschichtungsmittel nur unter Verwendung von Wasser als Lösungsmittel erfolgt, da solche wässrigen Beschichtungsmittel besonders vorteilhafte Umwelteigenschaften aufweisen.

Generell können den mit den erfindungsgemäßen hydrophilen Polyasparaginsäureestern oder deren, bevorzugt wässrigen, Lösungen oder Dispersionen formulierten, bevorzugt wässrigen, Lacken und Beschichtungsmitteln gegebenenfalls weitere auf dem Lacksektor üblichen Hilfs- und Zusatzmittel zugesetzt werden. Als Hilfs- und Zusatzmittel eignen sich hierbei beispielsweise Verlaufshilfsmittel, Farbpigmente, Füllstoffe, Mattierungsmittel, anorganische oder organische Pigmente, Lichtschutzmittel, Lackadditive, wie Dispergiermittel, Verlaufsmittel, Verdicker, Entschäumer und andere Hilfsmittel, Haftmittel, Fungizide, Bakterizide, Stabilisatoren oder Inhibitoren und Katalysatoren oder Emulgatoren.

Ein weiterer Gegenstand der Erfindung ist ein 2-Komponenten-System enthaltend eine Komponente a), umfassend wenigstens einen erfindungsgemäßen hydrophilen Polyasparaginsäureester oder wenigstens eine Lösung oder Dispersion der hydrophilen Polyasparaginsäureester und eine Komponente b), umfassend wenigstens ein NCO-funktionelles Vernetzungsmittel. Derartige Systeme bieten unter anderem den Vorteil, dass sich harte und elastische Beschichtungen erhalten lassen, die sich durch hervorragende Lösungsmittelbeständigkeiten auszeichnen.

Es ist hierbei weiterhin bevorzugt, das wenigstens ein weiterer Bestandteil der 2-Komponenten-Systeme Wasser ist, wobei das Wasser in der Komponente a) und/oder in der Komponente b) enthalten sein kann. Ein derartiges 2-Komponenten-System weist besonders vorteilhafte Umwelteigenschaften bei gleichbleibend sehr guten physiko-chemischen Eigenschaften auf.

Gemäß einer weiteren bevorzugten Ausführungsform kommen in dem 2-Komponenten-System oben genannte hydrophil modifizierte Polyisocyanate als NCO-funktionelle Vernetzungsmittel zum Einsatz.

In einer weiteren bevorzugten Ausführungsform können obige 2-Komponenten-Systeme unter Einsatz unblockierter NCO-funktioneller Vernetzungsmittel als Komponente b) bereits bei Raumtemperaturtrocknung hochglänzende, harte und elastische Beschichtungen ergeben, die sich durch hervorragende Lösungsmittelbeständigkeiten auszeichnen. Es ist jedoch ebenso möglich, dass diese 2-Komponenten-Systeme unter forcierten Bedingungen bei erhöhter Temperatur bzw. durch Einbrennen bei Temperaturen bis 260°C getrocknet werden. Werden nach einer anderen bevorzugten Ausführungsform blockierte NCO-funktionelle Vernetzungsmittel eingesetzt, können bis zur vollständigen Aushärtung Temperaturen von mindestens 60°C, bevorzugt von mindestens 80°C erforderlich sein.

Im Allgemeinen lassen sich die erfindungsgemäßen 2-Komponenten-Systeme hervorragend zur Herstellung einer Beschichtung auf einem Substrat verwenden.

Die Beschichtungsmittel basierend auf den Lösungen oder Dispersionen der erfindungsgemäßen hydrophilen Polyasparaginsäureester können nach bekannten Methoden wie beispielsweise durch Spritzen, Streichen, Fluten oder mit Hilfe von Walzen oder Rakeln auf beliebige Substrate aufgetragen werden. Als Substrate eignen sich beispielsweise Metall, Holz, Glas, Stein, keramische Materialien, Beton, harte und flexible Kunststoffe, Textilien, Leder oder Papier, die vor der Beschichtung gegebenenfalls auch mit üblichen Grundierungen versehen werden können.

Daher sind Substrate erhältlich durch Verwendung des erfindungsgemäßen 2-Komponenten-Systems ein weiterer Gegenstand der Erfindung.

### Beispiele

Die Erfindung wird im Folgenden an Hand von Beispielen näher erläutert.

Alle Prozentangaben beziehen sich, soweit nichts Anderslautendes vermerkt, auf das Gewicht.

Die Bestimmung der NCO-Gehalte erfolgte titrimetrisch nach DIN EN ISO 11909.

Die Aminzahlen wurden nach DIN 53176 durch Titration mit Salzsäure bestimmt.

Sämtliche Viskositätsmessungen erfolgten mit einem Physica MCR 51 Rheometer der Fa. Anton Paar Germany GmbH (DE) nach DIN EN ISO 3219.

Mittlere Teilchengrößen wässriger Dispersionen wurden mit einem Zetasizer, Typ DTS 5100, der Fa. Malvern Instruments GmbH (DE) bestimmt.

Die Messung der Hazen-Farbzahl erfolgte spektrofotometrisch nach DIN EN 1557 mit einem LICO 400-Spektrofotometer der Fa. Lange, DE.

Die Gehalte (mol-%) der in den Polyisocyanatkomponenten enthaltenen Allophanat-, Isocyanurat- und/oder Uretdionstrukturen wurden aus den Daten protokonenentkoppelter 13C-NMR-Spektren (aufgenommen an einem Gerät Bruker DPX-400) errechnet und beziehen sich jeweils auf die Summe an vorliegenden Allophanat-, Isocyanurat- und/oder Uretdionstrukturen. Die einzelnen Strukturelemente weisen im Falle von HDI-Polyisocyanaten folgende chemische Verschiebungen (in ppm) auf: Allophanat: 155.7 und 153.8; Isocyanurat: 148.4; Uretdion: 157.1.

### Ausgangsverbindungen

### Polyaminkomponente A)

### Polyaminkomponente A1)

Aminverbindung auf Basis von 4,4'-Diaminodicyclohexylmethan und Maleinsäurediethylester, hergestellt gemäß Beispiel b1) - I) der EP-A 0 403 921. Erhalten wurde ein klares Produkt mit folgenden Kennzahlen:

| | |
|---|---|
| Aminzahl: | 203 mg KOH/g |
| Äquivalentgewicht: | 276 g/val NH |
| Viskosität (23°C): | 1.450 mPas |

### Polyaminkomponente A2)

Aminverbindung auf Basis von 3,3'-Dimethyl-4,4'-diaminodicyclohexylmethan und Maleinsäurediethylester, hergestellt gemäß Beispiel b1) - D) der EP-A 0 403 921. Erhalten wurde ein farbloses, klares Produkt mit folgenden Kennzahlen:

| | |
|---|---|
| Aminzahl: | 193 mg KOH/g |
| Äquivalentgewicht: | 290 g/val NH |
| Viskosität (23°C): | 1.400 mPas |

### Polyisocyanatkomponente B)

### Polyisocyanatkomponente B1)

1344 g (8,0 mol) 1,6-Diisocyanatohexan (HDI) wurden bei einer Temperatur von 80°C unter trockenem Stickstoff mit 350 g (1,0 mol) eines auf Methanol gestarteten, monofunktionellen Polyethylenoxidpolyethers eines zahlenmittleren Molekulargewichtes von 350 g/mol versetzt und 3 Stunden gerührt, bis ein NCO-Gehalt von 37,2 %, entsprechend einer vollständigen Urethanisierung, erreicht war. Anschließend wurde das Reaktionsgemisch auf 90°C aufgeheizt und 0,2 g Zink(II)-2-ethyl-1-hexanoat als Allophanatisierungskatalysator zugegeben. Aufgrund der exotherm einsetzenden Reaktion stieg die Temperatur der Mischung bis auf 105°C an. Nach ca. 30 min betrug der NCO-Gehalt des Reaktionsgemisches 34,7 %. Der Katalysator wurde durch Zugabe von 0,2 g ortho-Phosphorsäure deaktiviert und das nicht umgesetzte monomere HDI bei einer Temperatur von 130°C und einem Druck von 0,1 mbar im Dünnschichtverdampfer abgetrennt. Man erhielt 670 g eines praktisch farblosen, klaren Polyether-modifizierten Polyisocyanates mit folgenden Kenndaten:

| | |
|---|---|
| NCO-Gehalt: | 11,4 % |
| monomeres HDI: | 0,04 % |
| Viskosität (23°C): | 148 mPas |
| NCO-Funktionalität: | 2,0 (berechnet) |
| Allophanatgruppen: | 97,0 mol-% |
| Isocyanuratgruppen: | 3,0 mol-% |

### Polyisocyanatkomponente B2)

Nach dem in Beispiel 1 beschriebenen Verfahren wurden 1344 g (8,0 mol) HDI mit 500 g (1,0 mol) eines auf Methanol gestarteten, monofunktionellen Polyethylenoxidpolyethers eines zahlenmittleren Molekulargewichtes von 500 g/mol umgesetzt. Die Allophanatisierungsreaktion wurde bei einem NCO-Gehalt von 34,2 % durch Zugabe von 0,2 g Zink(II)-2-ethyl-1-hexanoat gestartet. Nach Erreichen eines NCO-Gehaltes von 31,9 % wurde das Reaktionsgemisch mit 0,2 g ortho-Phosphorsäure gestoppt und wie in Beispiel 1 beschrieben aufgearbeitet. Man erhielt 740 g eines farblosen, klaren Polyether-modifizierten Polyisocyanates mit folgenden Kenndaten:

| | |
|---|---|
| NCO-Gehalt: | 9,0 % |
| monomeres HDI: | 0,06 % |
| Viskosität (23°C): | 197 mPas |
| NCO-Funktionalität: | 2,0 (berechnet) |
| Allophanatgruppen: | 97,0 mol-% |
| Isocyanuratgruppen: | 3,0 mol-% |

### Polyisocyanatkomponente B3)

Nach dem in Beispiel 1 beschriebenen Verfahren wurden 1344 g (8,0 mol) HDI mit 1400 g (1,0 mol) eines auf n-Butanol gestarteten monofunktionellen Ethylenoxid/Propylenoxid-Polyethers mit einem Ethylenoxidgehalt von 52 % und einem zahlenmittleren Molekulargewicht von 1400 g/mol umgesetzt. Die Allophanatisierungsreaktion wurde bei einem NCO-Gehalt von 22,9 % durch Zugabe von 0,3 g Zink(II)-2-ethyl-1-hexanoat gestartet. Nach Erreichen eines NCO-Gehaltes von 21,4 % wurde das Reaktionsgemisch mit 0,3 g ortho-Phosphorsäure gestoppt und wie in Beispiel 1 beschrieben aufgearbeitet. Man erhielt 1737 g eines farblosen, klaren Polyether-modifizierten Polyisocyanates mit folgenden Kenndaten:

| | |
|---|---|
| NCO-Gehalt: | 4,0 % |
| monomeres HDI: | 0,05 % |
| Viskosität (23°C): | 394 mPas |
| NCO-Funktionalität: | 2,0 (berechnet) |
| Allophanatgruppen: | 96,5 mol-% |
| Isocyanuratgruppen: | 3,5 mol-% |

### Polyisocyanatkomponente B4)

Nach dem in Beispiel 1 beschriebenen Verfahren wurden 1776 g (8,0 mol) Isophorondiisocyanat (IPDI) mit 500 g (1,0 mol) des bei der Herstellung der Polyisocyantkomponente B2) beschriebenen Polyetheralkohols umgesetzt. Die Allophanatisierungsreaktion wurde bei einem NCO-Gehalt von 27,7 % durch Zugabe von 0,3 g Zink(II)-2-ethyl-1-hexanoat gestartet. Nach Erreichen eines NCO-Gehaltes von 25,8 % wurde das Reaktionsgemisch mit 0,3 g ortho-Phosphorsäure gestoppt. Das nicht umgesetzte monomere IPDI wurde bei einer Temperatur von 160°C und einem Druck von 0,1 mbar im Dünnschichtverdampfer abgetrennt. Man erhielt 878 g eines schwach gelben, klaren Polyether-modifizierten Polyisocyanates mit folgenden Kenndaten:

| | |
|---|---|
| NCO-Gehalt: | 6,6 % |
| monomeres IPDI: | 0,05 % |
| Viskosität (23°C): | 386 mPas |
| NCO-Funktionalität: | 2,0 (berechnet) |
| Allophanatgruppen: | 98,5 mol-% |
| Isocyanuratgruppen: | 2,5 mol-% |

### Polyisocyanatkomponente B5)

Nach dem in Beispiel 1 beschriebenen Verfahren wurden 1776 g (8,0 mol) Isophorondiisocyanat (IPDI) mit 1400 g (1,0 mol) des bei der Herstellung der Polyisocyantkomponente B3) beschriebenen Polyetheralkohols umgesetzt. Die Allophanatisierungsreaktion wurde bei einem NCO-Gehalt von 19,8 % durch Zugabe von 0,3 g Zink(II)-2-ethyl-1-hexanoat gestartet. Nach Erreichen eines NCO-Gehaltes von 18,5 % wurde das Reaktionsgemisch mit 0,3 g ortho-Phosphorsäure gestoppt. Das nicht umgesetzte monomere IPDI wurde bei einer Temperatur von 160°C und einem Druck von 0,1 mbar im Dünnschichtverdampfer abgetrennt. Man erhielt 1810 g eines schwach gelben, klaren Polyether-modifizierten Polyisocyanates mit folgenden Kenndaten:

| | |
|---|---|
| NCO-Gehalt: | 3,4 % |
| monomeresIPDI: | 0,04 % |
| Viskosität (23°C): | 850 mPas |
| NCO-Funktionalität: | 2,0 (berechnet) |
| Allophanatgruppen: | 98,0 mol-% |
| Isocyanuratgruppen: | 2,0 mol-% |

### Polyisocyanatkomponente B6)

480 g (2,48 val) eines Isocyanuratgruppenhaltigen Polyisocyanates auf Basis von HDI mit einem NCO-Gehalt von 21,7 %, einer mittleren NCO-Funktionalität von 3,5 (nach GPC), einem Gehalt an monomerem HDI von 0,1 % und einer Viskosität von 3000 mPas (23 °C) wurden bei 100 °C unter trockenem Stickstoff und Rühren vorgelegt, innerhalb von 30 min mit 520 g (1,04 mol) eines auf Methanol gestarteten, monofunktionellen Polyethylenoxidpolyethers eines zahlenmittleren Molekulargewichtes von 500 g/mol versetzt und bei dieser Temperatur weiter gerührt, bis der NCO-Gehalt der Mischung nach etwa 2 h auf den einer vollständigen Urethanisierung entsprechenden Wert von 6,0 % gefallen war. Nach Abkühlen auf Raumtemperatur lag ein farbloses, klares Polyether-modifiziertes Polyisocyanatgemisch mit folgenden Kenndaten vor:

| | |
|---|---|
| NCO-Gehalt: | 6,0 % |
| monomeres HDI: | 0,04 % |
| Viskosität (23°C): | 4.310 mPas |
| NCO-Funktionalität: | 2,0 (berechnet) |

### Beispiel 1 (erfindungsgemäß)

900 g (3,26 mol) der Polyaminkomponente A1) wurden bei einer Temperatur von 40°C unter trockenem Stickstoff vorgelegt, mit 100g (0,27 val) der Polyisocyanatkomponente B1), entsprechend einem Äquivalentverhältnis von NH : NCO von 12,0 : 1, versetzt und ca. 2 h bis zum vollständigen Verschwinden der Isocyanatbande (bei ca. 2270 cm⁻¹) im IR-Spektrum gerührt. Der farbhelle, klare, erfindungsgemäße Polyasparaginsäureester wies folgende Kenndaten auf:

| | |
|---|---|
| Aminzahl: | 166 mg KOH/g |
| Äquivalentgewicht: | 337 g/val NH |
| Viskosität (23°C): | 1.400 mPas |
| Farbzahl (APHA): | 30 Hazen |

300 g dieses Polyasparaginsäureesters wurden in einem Becherglas vorgelegt, mit 200 g entionisiertem Wasser versetzt und durch einfaches Rühren mit einem Magnetrührer in eine stabile wässrige Dispersion mit einem Festkörpergehalt von 60 % überführt. Die mittlere Teilchengröße betrug 144 nm.

### Beispiel 2 bis 11 (erfindungsgemäß)

Nach dem in Beispiel 1 beschriebenen Verfahren wurden die Polyaminkomponenten A1) und A2) mit den Polyisocyanatkomponenten B1) bis B6) zu erfindungsgemäßen hydrophilen Polyasparaginsäureestern umgesetzt. Die nachfolgende Tabelle 1 zeigt die Zusammensetzungen der Reaktionsansätze (jeweils Gew.-Teile) sowie Kenndaten der erhaltenen erfindungsgemäßen hydrophilen Polyasparaginsäureester bzw. den aus den Polyasparaginsäureestern erhaltenen erfindungsgemäßen wässrigen Dispersionen. Die in den erfindungsgemäßen Beispielen 2 bis 11 erhaltenen hydrophilen Polyasparaginsäureester waren ebenfalls wie der nach dem erfindungsgemäßen Beispiel 1 erhaltene hydrophile Polyasparaginsäureester farbhell. Dies ist an den gemessenen Hazen-Farbzahlen (APHA) von 17 bis 58 Hazen erkennbar.

**Tabelle 1:**

| **Beispiel** | **2** | **3** | **4** | **5** | **6** | **7** | **8** | **9** | **10** | **11** |
|---|---|---|---|---|---|---|---|---|---|---|
| Polyaminkomponente A1) | 80 | 80 | 85 | 75 | - | - | - | - | - | 80 |
| Polyaminkomponente A2) | - | - | - | - | 80 | 80 | 80 | 80 | 80 | - |
| Polyisocyanatkomponente B1) | - | - | - | - | 20 | - | - | - | - | - |
| Polyisocyanatkomponente B2) | 20 | - | - | - | - | 20 | - | - | - | - |
| Polyisocyanatkomponente B3) | - | 20 | 15 | 25 | - | - | 20 | - | - | - |
| Polyisocyanatkomponente B4) | - | - | - | - | - | - | - | 20 | - | - |
| Polyisocyanatkomponente B5) | - | - | - | - | - | - | - | - | 20 | - |
| Polyisocyanatkomponente B6) | - | - | - | - | - | - | - | - | - | 20 |
| NH : NCO [val] | 6,8 : 1 | 15,2 : 1 | 21,6 : 1 | 11,3 : 1 | 5,1 : 1 | 6,4 : 1 | 14,5 : 1 | 8,8 : 1 | 17,0 : 1 | 10,1 : 1 |
| Farbzahl [Hazen] | 28 | 31 | 17 | 27 | 33 | 27 | 47 | 33 | 58 | 22 |
| Aminzahl [mg KOH/g] | 137 | 150 | 163 | 136 | 123 | 131 | 142 | 133 | 143 | 147 |
| Äquivalentgewicht [g/val NH] | 409 | 373 | 344 | 412 | 455 | 427 | 394 | 421 | 392 | 381 |
| Viskosität (23°C) [mPas] | 1.380 | 2.410 | 2.270 | 2.590 | 1.880 | 7.700 | 2.850 | 16.200 | 3.940 | 5.440 |
| MTG [nm] ^{a)} | 86 | 208 | 546 | 141 | 134 | 81 | 241 | 151 | 250 | 488 |

| | | | | | | | | | | |
|---|---|---|---|---|---|---|---|---|---|---|
| ^{a)} MTG = mittlere Teilchengröße einer 60 %igen wässrigen Dispersion | | | | | | | | | | |

### Beispiel 12 (erfindungsgemäß)

27 Gew.-Teile des erfindungsgemäßen hydrophilen Polyasparaginsäureesters aus Beispiel 8 wurden nach dem in Beispiel 1 beschriebenen Verfahren mit 18 g entionisiertem Wasser zu einer 60 %igen wässrigen Dispersion verarbeitet, mit 0,15 Gew.-Teilen BYK®-349 und 0,35 Gew.-Teilen BYK®-378 (Silikontenside) versetzt und anschließend mit weiteren 32,25 Gew.-Teilen entionisiertem Wasser auf einen Festkörpergehalt von 35 % eingestellt. Diesem Ansatz wurden 13,6 Gew.-Teile eines von der Fa. Bayer MaterialScience (DE) unter der Bezeichnung Bayhydur® XP 2655 als Härter für wässrige 2K-Polyurethansysteme vermarkteten anionisch hydrophilierten HDI-Polyisocyanates mit einem NCO-Gehalt von 21,2 % und einer Viskosität (23°C) von 3.500 mPas in Form einer 80 %igen Lösung in 3-Methoxy-n-butylacetat (Gesamtzugabemenge: 17,0 Gew.-Teile) zugesetzt (entsprechend einem Äquivalentverhältnis von Isocyanatgruppen zu Aminogruppen von 1:1) und die Mischung durch intensives Rühren (2000 U/min) homogenisiert.

Analog wurde aus 27 Gew.-Teilen des Polyasparaginsäureesters aus Beispiel 10 unter Verwendung von Bayhydur® XP 2655 als Vernetzer (Äquivalentverhältnis Isocyanatgruppen:Aminogruppen 1:1) ein weiterer wässriger Lackansatz formuliert. Die Verarbeitungszeit der applikationsfertigen Lacke betrug etwa 3 Stunden. Die Lacke wurden in einer Naßfilm-Schichtdicke von 120 µm (ca. 55 µm trocken) auf Glasplatten appliziert und bei Raumtemperatur getrocknet.

Es wurden farblose, glänzende Lackfilme erhalten, die nach einem Tag folgende Eigenschaften aufwiesen:

| **Polyasparaginsäureester aus Beispiel** | **8** | **10** |
|---|---|---|
| Pendelhärte [s] nach 1 d / 7 d ^{a)} | 61 / 134 | 87/ 210 |
| Lösungsmittelbeständigkeit nach 7 d ^{b)} | | |
| Xylol | 2 | 1 |
| Ethylacetat | 2 | 1 |
| Methoxypropylacetat | 2 | 1 |
| Aceton | 5 | 5 |

| | | |
|---|---|---|
| ^{a)} Pendelhärte nach König (DIN 53157) ^{b)} Einwirkzeit jeweils 1 min | | |

### Bewertung:

0 = Lackoberfläche ist unverändert
1 = Lackoberfläche weist bleibende Markierung (Rand) auf
2 = Lackoberfläche ist mit dem Fingernagel ankratzbar
3 = Lackoberfläche ist mit dem Fingernagel teilweise abkratzbar
4 = Lackoberfläche ist mit dem Fingernagel abkratzbar bis zum Untergrund
5 = Lackoberfläche ist völlig zerstört

Die erfindungsgemäßen Beispiele 1 bis 11 zeigen, dass die hydrophilen Polyasparaginsäureester Hazen-Farbzahlen von 17 bis 58 Hazen aufweisen. Die aus diesen erfindungsgemäßen hydrophilen Polyasparaginsäureestern erhältlichen Beschichtungen sind farblos, was beispielhaft durch das Anwendungsbeispiel 12 gezeigt wurde. Neben der Eigenschaft, dass auch die erhaltenen Beschichtungen farblos sein können, besitzen die Beschichtungen aus dem Anwendungsbeispiel 12 zusätzlich eine sehr gute mechanische Stabilität und Lösemittelbeständigkeit.

## Patentansprüche

1. Hydrophile Polyasparaginsäureester, erhältlich durch Umsetzung von
wenigstens einer Polyaminkomponente A), umfassend eine Aminverbindung der Formel (I), 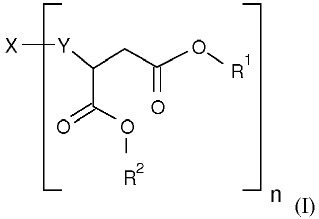 in welcher
X ein gesättigter oder ungesättigter, linearer oder verzweigter, aliphatischer oder cycloaliphatischer oder aromatischer organischer Rest ist, der substituiert oder unsubstituiert ist und/oder Heteroatome in der Kette aufweist,
Y eine an zwei Kohlenstoffatome gebundene sekundäre Aminogruppe ist,
R¹ und R² unabhängig voneinander gesättigte oder ungesättigte, lineare oder verzweigte, aliphatische oder cycloaliphatische oder aromatische organische Reste mit 1 bis 18 Kohlenstoffatomen sind, die substituiert oder unsubstituiert sind und/oder Heteroatome in der Kette aufweisen und
n eine natürliche Zahl von 1 bis 4 ist,
mit wenigstens einer Polyisocyanatkomponente B), umfassend ein Polyisocyanat, das wenigstens einen chemisch gebundenen Polyalkylenoxidpolyetheralkohol enthält, wobei die Anzahl der sekundären Aminogruppen Y zu der Anzahl der Isocyanatgruppen des Polyisocyanates in einem Verhältnis von 250:1 bis 3:1 steht.

2. Hydrophile Polyasparaginsäureester gemäß Anspruch 1, wobei die Anzahl der sekundären Aminogruppen Y in der Formel (I) zu der Anzahl der Isocyanatgruppen des Polyisocyanates in einem Verhältnis von 200:1 bis 5:1, bevorzugt 150:1 bis 6:1 und besonders bevorzugt 120:1 bis 8:1 steht.

3. Hydrophile Polyasparaginsäureester gemäß einem der Ansprüche 1 oder 2, wobei die chemisch gebundene, nicht-ionische, hydrophile Gruppe wenigstens eine Polyalkylenoxidpolyethereinheit umfasst, die zu ≥ 40 mol-%, bevorzugt zu ≥ 50 mol-% und besonders bevorzugt zu ≥ 70 mol-% aus Ethylenoxideinheiten besteht und/oder im statistischen Mittel 5 bis 80, bevorzugt 5 bis 50 und besonders bevorzugt 5 bis 30 Ethylenoxideinheiten aufweist.

4. Hydrophile Polyasparaginsäureester gemäß einem der Ansprüche 1 bis 3, wobei das Polyisocyanat wenigstens ein aliphatisches, cycloaliphatisches, araliphatisches oder aromatisches, bevorzugt wenigstens ein aliphatisches oder cycloaliphatisches Polyisocyanat ist.

5. Hydrophile Polyasparaginsäureester gemäß einem der Ansprüche 1 bis 4, wobei das Polyisocyanat Allophanatgruppen enthält und gegebenenfalls bis zu 10 mol-%, bevorzugt bis zu 5 mol-%, an Isocyanurat- und/oder Uretdiongruppen, bezogen auf die Gesamtmenge an Allophanat-, Isocyanurat- und Uretdionstrukturen, aufweist.

6. Hydrophile Polyasparaginsäureester gemäß einem der Ansprüche 1 bis 5, wobei in der Aminverbindung
X ein organischer Rest mit einem Molekulargewicht von 28 g/mol bis 6000 g/mol, bevorzugt 45 bis 5000 g/mol und besonders bevorzugt 84 bis 500 g/mol ist.

7. Hydrophile Polyasparaginsäureester gemäß einem der Ansprüche 1 bis 6, wobei in der Aminverbindung
X aus einer Gruppe von organischen Resten ausgewählt ist, die erhalten wird, wenn die Aminogruppen der Verbindungen 1,5-Diamino-2-methylpentan, 2,4'-Diamino-dicyclohexylmethan, 4,4'-Diamino-dicyclo-hexylmethan, 3,3'-Dimethyl-4,4'-diamino-dicyclohexylmethan oder Polypropylenglykoldiaminen des Molekulargewichtsbereiches 200 bis 500 g/mol entfernt werden und
n die natürliche Zahl 2 ist.

8. Hydrophile Polyasparaginsäureester gemäß einem der Ansprüche 1 bis 7, wobei in der Aminverbindung
R¹ und R² unabhängig voneinander aliphatische organische Reste mit 1 bis 9 Kohlenstoffatomen, bevorzugt unabhängig voneinander Methyl-, Ethyl-, n-Butyl- und/oder 2-Ethylhexylreste und besonders bevorzugt Ethylreste sind.

9. Lösungen oder Dispersionen enthaltend wenigstens einen hydrophilen Polyasparaginsäureester gemäß einem der Ansprüche 1 bis 8.

10. Lösungen oder Dispersionen gemäß Anspruch 9, wobei Wasser als Lösungsmittel enthalten ist und bevorzugt Wasser das alleinige Lösungsmittel ist.

11. Polyurethanharnstoff erhältlich durch Umsetzung wenigstens eines hydrophilen Polyasparaginsäureesters gemäß einem der Ansprüche 1 bis 8 mit wenigstens einem NCO-funktionellen Vernetzungsmittel.

12. Verfahren zur Herstellung eines Beschichtungsmittels, bei dem wenigstens ein hydrophiler Polyasparaginsäureester gemäß einem der Ansprüche 1 bis 8 oder wenigstens eine Lösung oder Dispersion gemäß einem der Ansprüche 9 oder 10 mit wenigstens einem NCO-funktionellen Vernetzungsmittel vermischt wird.

13. 2-Komponenten-System enthaltend eine Komponente a), umfassend wenigstens einen hydrophilen Polyasparaginsäureester gemäß einem der Ansprüche 1 bis 8 oder wenigstens eine Lösung oder Dispersion gemäß einem der Ansprüche 9 oder 10, und eine Komponente b), umfassend wenigstens ein NCO-funktionelles Vernetzungsmittel.

14. Verwendung des 2-Komponenten-Systems gemäß Anspruch 13 zur Herstellung von einer Beschichtung auf einem Substrat.

15. Substrate beschichtet mit dem 2-Komponenten-System gemäß Anspruch 13.

## Claims

1. Hydrophilic polyaspartic esters obtainable by reaction of at least one polyamine component A), comprising an amine compound of the formula (I), 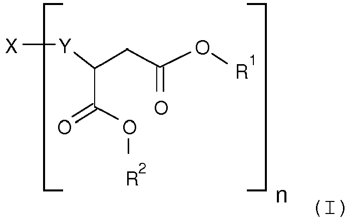 in which
X is a saturated or unsaturated, linear or branched, aliphatic or cycloaliphatic or aromatic organic radical which is substituted or unsubstituted and/or has heteroatoms in the chain,
Y is a secondary amino group bonded to two carbon atoms,
R¹ and R² independently of one another are saturated or unsaturated, linear or branched, aliphatic or cycloaliphatic or aromatic organic radicals having 1 to 18 carbon atoms, and are substituted or unsubstituted and/or have heteroatoms in the chain, and
n is a natural number from 1 to 4,
with at least one polyisocyanate component B), comprising a polyisocyanate which contains at least one chemically bonded polyalkylene oxide polyether alcohol, the ratio of the number of secondary amino groups Y to the number of isocyanate groups in the polyisocyanate being from 250:1 to 3:1.

2. Hydrophilic polyaspartic esters according to Claim 1, the ratio of the number of secondary amino groups Y in the formula (I) to the number of isocyanate groups in the polyisocyanate being from 200:1 to 5:1, preferably 150:1 to 6:1 and more preferably 120:1 to 8:1.

3. Hydrophilic polyaspartic esters according to either of Claims 1 and 2, the chemically bonded, non-ionic, hydrophilic group comprising at least one polyalkylene oxide polyether unit which consists to an extent of ≥ 40 mol%, preferably ≥ 50 mol% and more preferably ≥ 70 mol% of ethylene oxide units and/or which has on average 5 to 80, preferably 5 to 50 and more preferably 5 to 30 ethylene oxide units.

4. Hydrophilic polyaspartic esters according to any of Claims 1 to 3, the polyisocyanate being at least one aliphatic, cycloaliphatic, araliphatic or aromatic, preferably at least one aliphatic or cycloaliphatic, polyisocyanate.

5. Hydrophilic polyaspartic esters according to any of Claims 1 to 4, the polyisocyanate containing allophanate groups and having optionally up to 10 mol%, preferably up to 5 mol%, of isocyanurate groups and/or uretdione groups, based on the total amount of allophanate, isocyanurate and uretdione structures.

6. Hydrophilic polyaspartic esters according to any of Claims 1 to 5, where in the amine compound X is an organic radical having a molecular weight of 28 g/mol to 6000 g/mol, preferably 45 to 5000 g/mol and more preferably 84 to 500 g/mol.

7. Hydrophilic polyaspartic esters according to any of Claims 1 to 6, where in the amine compound
X is selected from a group of organic radicals obtained when the amino groups of the compounds 1,5-diamino-2-methylpentane, 2,4'-diamino-dicyclohexylmethane, 4,4'-diaminodicyclohexylmethane, 3,3'-dimethyl-4,4'-diaminodicyclohexylmethane or polypropylene glycol diamines of the molecular weight range 200 to 500 g/mol are removed, and
n is the natural number 2.

8. Hydrophilic polyaspartic esters according to any of Claims 1 to 7, where in the amine compound
R¹ and R² independently of one another are aliphatic organic radicals having 1 to 9 carbon atoms, preferably independently of one another methyl, ethyl, n-butyl and/or 2-ethylhexyl radicals and more preferably ethyl radicals.

9. Solutions or dispersions comprising at least one hydrophilic polyaspartic ester according to any of Claims 1 to 8.

10. Solutions or dispersions according to Claim 9, where water is present as solvent, and preferably water is the sole solvent.

11. Polyurethaneurea obtainable by reaction of at least one hydrophilic polyaspartic ester according to any of Claims 1 to 8 with at least one NCO-functional crosslinking agent.

12. Method for producing a coating composition, wherein at least one hydrophilic polyaspartic ester according to any of Claims 1 to 8 or at least one solution or dispersion according to either of Claims 9 and 10 is mixed with at least one NCO-functional crosslinking agent.

13. 2-Component system comprising a component a), comprising at least one hydrophilic polyaspartic ester according to any of Claims 1 to 8 or at least one solution or dispersion according to either of Claims 9 and 10, and a component b), comprising at least one NCO-functional crosslinking agent.

14. Use of the 2-component system according to Claim 13 for producing a coating on a substrate.

15. Substrates coated with the 2-component system according to Claim 13.

## Revendications

1. Poly(ester de l'acide aspartique) hydrophile, pouvant être obtenu par transformation d'au moins un composant de type polyamine A), comprenant un composé de type amine de formule (I) 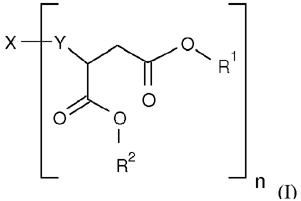 dans laquelle
X représente un radical organique saturé ou insaturé, linéaire ou ramifié, aliphatique ou cycloaliphatique ou aromatique, qui est substitué ou non substitué et/ou qui présente des hétéroatomes dans la chaîne,
Y représente un groupe amino secondaire lié à deux atomes de carbone,
R¹ et R² représentent, indépendamment l'un de l'autre, des radicaux organiques saturés ou insaturés, linéaires ou ramifiés, aliphatiques ou cycloaliphatiques ou aromatiques, comprenant 1 à 18 atomes de carbone, qui sont substitués ou non substitués et/ou qui présentent des hétéroatomes dans la chaîne et
n représente un nombre naturel de 1 à 4,
avec au moins un composant de type polyisocyanate B), comprenant un polyisocyanate qui contient au moins un poly(oxyde d'alkylène)polyétheralcool lié chimiquement, le nombre de groupes amino secondaire Y au nombre de groupes isocyanate du polyisocyanate se trouvant dans un rapport de 250:1 à 3:1.

2. Poly(ester de l'acide aspartique) hydrophile selon la revendication 1, le nombre de groupes amino secondaire Y dans la formule (I) au nombre de groupes isocyanate du polyisocyanate se trouvant dans un rapport de 200:1 à 5:1, de préférence de 150:1 à 6:1 et de manière particulièrement préférée de 120:1 à 8:1.

3. Poly(ester de l'acide aspartique) hydrophile selon l'une quelconque des revendications 1 ou 2, le groupe hydrophile, non ionique, lié chimiquement comprenant au moins une unité poly(oxyde d'alkylène)-polyéther, qui est constituée à raison de ≥ 40% en mole, de préférence à raison de ≥ 50% en mole et de manière particulièrement préférée à raison de ≥ 70% en mole d'unités d'oxyde d'éthylène et/ou qui présente en moyenne statistique 5 à 80, de préférence 5 à 50 et de manière particulièrement préférée 5 à 30 unités d'oxyde d'éthylène.

4. Poly(ester de l'acide aspartique) hydrophile selon l'une quelconque des revendications 1 à 3, le polyisocyanate étant au moins un polyisocyanate aliphatique, cycloaliphatique, araliphatique ou aromatique, de préférence au moins un polyisocyanate aliphatique ou cycloaliphatique.

5. Poly(ester de l'acide aspartique) hydrophile selon l'une quelconque des revendications 1 à 4, le polyisocyanate contenant des groupes allophanate et présentant le cas échéant jusqu'à 10% en mole, de préférence jusqu'à 5% en mole, de groupes isocyanurate et/ou uretdione, par rapport à la quantité totale de structures allophanate, isocyanurate et uretdione.

6. Poly(ester de l'acide aspartique) hydrophile selon l'une quelconque des revendications 1 à 5, où, dans le composé de type amine,
X représente un radical organique présentant un poids moléculaire de 28 g/mole à 6000 g/mole, de préférence de 45 à 5000 g/mole et de manière particulièrement préférée de 84 à 500 g/mole.

7. Poly(ester de l'acide aspartique) hydrophile selon l'une quelconque des revendications 1 à 6, où, dans le composé de type amine,
X est choisi dans un groupe de radicaux organiques qui est obtenu lorsque les groupes amino des composés 1,5-diamino-2-méthylpentane, 2,4'-diaminodicyclohexylméthane, 4,4'-diaminodicyclohexylméthane, 3,3'-diméthyl-4,4'-diaminodicyclohexylméthane ou polypropylèneglycoldiamines de la plage de poids moléculaires de 200 à 500 g/mole sont éliminés et
n vaut le nombre naturel 2.

8. Poly(ester de l'acide aspartique) hydrophile selon l'une quelconque des revendications 1 à 7, où, dans le composé de type amine,
R¹ et R² représentent, indépendamment l'un de l'autre, des radicaux organiques aliphatiques comprenant 1 à 9 atomes de carbone, de préférence indépendamment l'un de l'autre des radicaux méthyle, éthyle, n-butyle et/ou 2-éthylhexyle et de manière particulièrement préférée des radicaux éthyle.

9. Solutions ou dispersions contenant au moins un poly(ester de l'acide aspartique) hydrophile selon l'une quelconque des revendications 1 à 8.

10. Solutions ou dispersions selon la revendication 9, de l'eau étant contenue comme solvant et l'eau étant de préférence le seul solvant.

11. Polyuréthane-urée pouvant être obtenue par transformation d'au moins un poly(ester de l'acide aspartique) hydrophile selon l'une quelconque des revendications 1 à 8 avec au moins un réticulant à fonctionnalité NCO.

12. Procédé pour la réalisation d'un agent de revêtement, dans lequel au moins un poly(ester de l'acide aspartique) hydrophile selon l'une quelconque des revendications 1 à 8 ou au moins une solution ou dispersion selon l'une quelconque des revendications 9 ou 10 est mélangé(e) avec au moins un réticulant à fonctionnalité NCO.

13. Système à 2 composants contenant un composant a), comprenant au moins un poly(ester de l'acide aspartique) hydrophile selon l'une quelconque des revendications 1 à 8 ou au moins une solution ou dispersion selon l'une quelconque des revendications 9 ou 10, et un composant b), comprenant au moins un réticulant à fonctionnalité NCO.

14. Utilisation du système à 2 composants selon la revendication 13 pour la réalisation d'un revêtement sur un substrat.

15. Substrats revêtus par le système à 2 composants selon la revendication 13.
